# FASCICULE DE BREVET EUROPEEN

(11) **EP 2 739 275 B1**
(45) Date de publication et mention de la délivrance du brevet: **16.01.2019**
(21) Numéro de dépôt: 12750471.0
(22) Date de dépôt: 02.08.2012
(51) Int. Cl.: A61K 31/07, A61K 31/355, A61K 31/722, A61K 31/728, A61K 33/38, A61K 45/06, A61K 9/00, A61P 17/02, A61K 31/38, A61K 31/216, A61K 31/715, A61K 33/30, A61L 26/00, A61K 47/02, A61K 47/36, A61K 9/107

(54) **COMPOSITION ANTISEPTIQUE**
ANTISEPTISCHE ZUSAMMENSETZUNG
ANTISEPTIC COMPOSITION

(30) Priorité: 04.08.2011 FR 1157144
(43) Date de publication de la demande: 11.06.2014
(73) Titulaire: Petcare Innovation, 59118 Wambrechies (FR)
(72) Inventeur: ALLART, Jean-Claude, 62520 Le Touquet (FR); LEFEVRE, Jean-Marie, 80000 Amiens (FR); PEYROT, Jacques, 63000 Clermont-Ferrand (FR)
(74) Mandataire: Peguet, Wilfried
(86) Numéro de dépôt international: PCT/FR2012/051838
(87) Numéro de publication internationale: WO 2013/017807

(56) Documents cités:
- EP-A1- 2 371 350
- EP-A2- 0 138 572
- WO-A1-2004/112805
- WO-A1-2009/036714
- WO-A1-2010/127647
- WO-A1-2012/066447
- WO-A1-2013/017808
- DE-A1-102005 008 299
- DE-A1-102007 044 202
- DE-A1-102007 044 583
- US-A1- 2003 175 332
- MUZZARELLI R A A ET AL: "BIOCHEMISTRY, HISTOLOGY AND CLINICAL USES OF CHITINS AND CHITOSANS IN WOUND HEALING", EXS, BIRKHAEUSER VERLAG, BASEL, CH, vol. 87, 1 janvier 1999 (1999-01-01), pages 251-264, XP008019129, ISSN: 1023-294X
- OKAMOTO Y ET AL: "Application of polymeric N-acetyl-D-glucosamine (chitin) to veterinary practice", JOURNAL OF VETERINARY MEDICAL SCIENCE - NIHON JUIGAKU ZASSHI, JAPANESE SOCIETY OF VETERINARY SCIENCE, TOKYO, JP, vol. 55, no. 5, 1 octobre 1993 (1993-10-01), pages 743-747, XP002670010, ISSN: 0916-7250
- ANONYMOUS: "Sansil animal - Die Wundheilpflege - Creme - 50 ml", INTERNET CITATION, 6 octobre 2010 (2010-10-06), pages 1-4, XP002670011, Extrait de l'Internet: URL:http://web.archive.org/web/20101006091 323/http://www.hundeshop.de/gesundheit/san sil-animal--die-wundheil-und-pflegecreme-m it-silber.php [extrait le 2012-02-20]
- PIRNAZAR P ET AL: "Bacteriostatic effects of hyaluronic acid", JOURNAL OF PERIODONTOLOGY 199904 US, vol. 70, no. 4, April 1999 (1999-04), pages 370-374, XP008162701, ISSN: 0022-3492
- NAKAMURA M ET AL: "Concentration and molecular weight dependency of rabbit corneal epithelial wound healing on hyaluronan.", CURRENT EYE RESEARCH OCT 1992, vol. 11, no. 10, October 1992 (1992-10), pages 981-986, XP008097255, ISSN: 0271-3683

## Description

La présente invention concerne une nouvelle composition à propriétés antiseptique et cicatrisante, et plus particulièrement une nouvelle composition dermatologique à base d'argent et d'acide hyaluronique, utile notamment en médecine vétérinaire, pour le traitement des plaies cutanées et leur cicatrisation.

La peau constitue à la fois une barrière anatomique vivante et une zone d'échange entre le corps et son environnement, dont l'efficacité conditionne le maintien d'un bon équilibre homéostasique. La peau est un véritable organe comprenant plusieurs couches intégrées, allant de la couche superficielle, l'épiderme, jusqu'aux couches plus profondes, le derme et l'hypoderme, où chacune de ces couches remplit des fonctions permettant à l'ensemble de réagir et de s'adapter à son environnement.

L'épiderme, principalement composé de kératinocytes, de mélanocytes et des cellules de Langerhans, a une épaisseur variable selon les différentes parties du corps, et constitue la couche externe de la peau pour assurer la protection du corps vis-à-vis de son environnement extérieur. Le derme est la couche la plus épaisse, traversée par des fibres nerveuses et des vaisseaux sanguins, se composant principalement de collagène, d'élastine, de protéoglycanes et de glycosaminoglycanes, principalement synthétisés par les fibroblastes dermiques. Les fibres de collagène assurent la résistance mécanique et la texture de la peau, l'élastine est responsable de son élasticité, les glycosaminoglycanes et les protéoglycanes jouent un rôle majeur de structure et d'hydratation de la peau. La couche la plus profonde de la peau forme l'hypoderme, contenant les adipocytes qui produisent des lipides assurant la formation d'une couche grasse protégeant les muscles, les os et les organes internes contre les chocs.

Chez l'animal tel que le cheval, le chien et le chat, la peau comprend aussi des follicules pilo-sébacés très développés qui jouent un rôle important dans la protection et l'équilibre homéostasique. La présence du pelage, qui doit être lubrifié et protégé, explique la fonction importante de la glande sébacée dans l'équilibre physiologique de l'animal. Les troubles de la kératinisation (génétiques ou acquis liés au conditions de vie de l'animal), fréquents chez les animaux de compagnie, s'accompagnent d'une fragilisation du pelage et du développement de surpopulation bactérienne et fongique. De plus, les infections primitives, les surinfections de dermatoses et de plaies, et les parasitoses, sont fréquentes. Certaines zones anatomiques chez le chat et le chien, comme les oreilles, l'oeil, les zones de jonction muqueuse-peau et les coussinets, sont exposées et plus souvent atteintes par une infection.

Les plaies cutanées, chez l'animal comme chez l'homme, induisent des phénomènes inflammatoires de cicatrisation qui peuvent être perturbés par l'hypoxie aiguë engendrée par le traumatisme et par la pullulation microbienne déclenchée par l'effraction cutanée. Une surpopulation bactérienne a pour effet de modifier les conditions de la cicatrisation en la retardant ou en la bloquant, et en favorisant la diffusion d'une infection à partir de la plaie initiale.

La plupart des colonies bactériennes se transforment en biofilms, structure membranaire de survie microbienne, détournant les facteurs de croissance et de cicatrisation en créant une inflammation qui empêche le processus de défense antimicrobienne d'agir efficacement. Les bactéries des biofilms résistent à la plupart des antibiotiques et des antiseptiques, et il est donc nécessaire de mettre au point des compositions susceptibles de gérer la flore microbienne et de faciliter la cicatrisation des plaies.

L'argent est connu comme antiseptique et a été utilisé pendant des années, notamment dans des crèmes pour traiter des infections néonatales et plus largement comme antiseptique efficace contre les bactéries Gram positif et Gram négatif, aérobies et anaérobies. La forme active est l'ion argent Ag⁺ qui peut être apporté par divers sels et complexes d'argent tels que le nitrate, le chlorure, ou le thiosulfate d'argent, ou encore divers carboxylates tels que l'acétate, ou des complexes dérivés de sulfamides comme la sulfadiazine argentique. Des exemples de sels d'argent à propriétés antimicrobiennes sont décrits dans la demande de brevet US 2006051385.

Ces sels d'argent ont été utilisés car ils sont relativement faciles à formuler sous forme pharmaceutique et leur prix de revient est généralement peu élevé. Toutefois, ils présentent une toxicité par résorption systémique qui limite leur emploi, sinon à faible concentration, ce qui limite fortement leur efficacité. L'argent peut aussi être utilisé sous forme métallique mais le prix de revient de compositions comprenant de l'argent métallique est alors très élevé, et, de plus, la solubilisation de l'argent métallique est très difficile. Toutefois, bien que les compositions connues à base d'argent, telles que la sulfadiazine argentique, présentent souvent une bonne tolérance cutanée, elles n'ont pas d'avantage bactéricide par rapport aux antiseptiques de référence tels que la chlorhexidine, notamment sur les bactéries Gram négatif. Le brevet EP 241175 décrit l'association de sulfadiazine argentique avec du miconazole ou du clotrimazole dans une composition antimicrobienne à plus large spectre. La chlorhexidine et la sulfadiazine argentique ont aussi été proposées en association, plus particulièrement comme antiseptique en milieu hospitalier pour la prévention d'infections, sans procurer d'avantage significatif (Hockenhull JC et al., Health Technol. Assess. 2008:12).

L'acide hyaluronique est un polymère naturel à base de disaccharide comprenant des unités acide D-glucuronique et N-acétylglucosamine liées par liaison glucosidique. Ses propriétés sont très variables selon son poids moléculaire. Les acides hyaluroniques à haut poids moléculaire, supérieur à 1.000 kDa, sont essentiellement utilisés pour favoriser l'hydratation de la peau grâce au réseau glucidique hydrophile qu'ils constituent. Les acides hyaluroniques de faible poids moléculaire, inférieur à 50 kDa environ, peuvent franchir la barrière du stratum corneum et stimulent les récepteurs CD44 responsables de la néosynthèse de l'acide hyaluronique dans le derme.

L'acide hyaluronique, comme le collagène, est un des constituants principaux de la matrice extracellulaire du derme et il joue un rôle important dans la croissance cellulaire et dans le maintien de l'hydratation. Au cours du vieillissement de la peau, on observe une diminution de sa concentration dans le derme. L'acide hyaluronique est un glycosaminoglycane souvent utilisé dans des compositions cosmétiques et dermatologiques, généralement sous forme de hyaluronate de sodium, notamment pour favoriser l'hydratation, et pour stimuler la cicatrisation et les défenses naturelles de la peau. Il est également utilisé en chirurgie esthétique pour le comblement des rides, dans des traitements médicaux contre l'arthrose, et en ophtalmologie. Divers dérivés d'acide hyaluronique, réticulés ou non réticulés, susceptibles de présenter une bonne résistance à la dégradation enzymatique, et utilisables en particulier dans des compositions cosmétiques, sont décrits par exemple dans la demande WO 2011080450.

On a aussi proposé d'associer l'acide hyaluronique ou des dérivés avec le chondroïtine sulfate, comme dans la demande WO 2009073437, avec un dérivé C-glycoside comme dans la demande WO 2009024719, ou avec un rétinoïde et un oligo-saccharide comme dans le brevet FR 2.894.827. Des sels de métaux lourds d'acide hyaluronique ont aussi été proposés dans des compositions antimicrobiennes, comme décrit dans la demande WO 8705517.

Les brevets DE 102007044583 et DE 102007044202 décrivent des compositions associant de l'argent colloïdal à de l'urée, éventuellement additionnées d'acide hyaluronique ou de hyaluronate de sodium, pour le traitement des plaies cutanées. Le brevet DE 102005008299 concerne une composition comprenant de l'argent, en tant qu'antiseptique, en association avec un hydratant tel que le Panthenol et de l'acide hyaluronique. Cependant aucun de ces documents ne donne de précision quant aux caractéristiques de l'acide hyaluronique, en particulier son poids moléculaire.

EP0138572 et US 2003175332 divulguent l'utilisation d'acide hyaluronique de poids moléculaire compris entre 100 et 500 kDa pour le traitement de plaies cutanées, y compris en médecine vétérinaire.

L'objet de la présente invention est de mettre au point une nouvelle composition topique qui présente des propriétés antiseptique et cicatrisante permettant de traiter efficacement les plaies cutanées contre la prolifération microbienne et de favoriser la cicatrisation, plus particulièrement chez l'animal.

La présente invention a donc pour objet une composition dermatologique pour utilisation en médecine vétérinaire, comprenant en association au moins de l'argent métallique sous forme de particules métalliques de dimension comprise entre 1 et 20 µm, et de l'acide hyaluronique ou un de ses sels de poids moléculaire compris entre 100 kDa et 500 kDa.

La présente invention a encore pour objet une composition dermatologique topique, antiseptique et cicatrisante, comprenant en association au moins de l'argent métallique sous forme de particules métalliques de dimension comprise entre 1 et 20 µm, et de l'acide hyaluronique ou un de ses sels de poids moléculaire compris entre 100 kDa et 500 kDa.

Enfin, la présente invention a pour objet une composition comprenant au moins de l'argent métallique sous forme de particules métalliques de dimension comprise entre 1 et 20 µm, et de l'acide hyaluronique ou un de ses sels de poids moléculaire compris entre 100 kDa et 500 kDa, en association, pour utilisation dans le traitement des plaies cutanées et leur cicatrisation, plus particulièrement en médecine vétérinaire.

L'argent métallique est sous forme micronisée, facilitant la dispersion dans une composition qui peut être hydrophile ou lipophile.

Suivant une forme avantageuse de réalisation, la composition de l'invention comprend en outre un dérivé d'acé-tylglucosamine choisi parmi la chitine et le chitosane.

Une telle composition est particulièrement utile en dermatologie vétérinaire pour le traitement de plaies cutanées grâce à ses propriétés antiseptiques et cicatrisantes.

L'argent métallique sous forme micronisée peut être aisément dispersé dans la composition de l'invention, qui peut être hydrophile ou lipophile, à des concentrations suffisantes pour procurer une bonne efficacité antiseptique. Les essais effectués avec les compositions de l'invention ont mis en évidence un spectre d'action antimicrobienne très large, avec une action spécifique sur les bactéries anaérobies des biofilms, que ne permettent pas d'atteindre les sels d'argents antimicrobiens connus, même en association avec un antiseptique tel que la chlorhexidine. La concentration en argent est généralement comprise entre 0,001% et 1,0% en poids par rapport au poids total de la composition.

L'argent métallique utilisé dans l'invention est avantageusement sous forme micronisée, la dimension des particules étant 1 à 20 µm, de pureté supérieure à 99,5%, et de préférence supérieure à 99,8%, présentant une activité anti-bactérienne et antimicrobienne efficace. Une forme d'argent métallique appropriée est constituée par une structure poreuse libérant progressivement les ions argent, comme par exemple la structure de micro-éponge du produit disponible dans le commerce sous la marque MicroSilver BG.

Suivant la présente description, par acide hyaluronique, on entend l'acide hyaluronique sous forme libre ou d'un de ses sels de métaux alcalins et alcalino-terreux, par exemple le hyaluronate de sodium, de potassium, de calcium ou de magnésium, de poids moléculaire moyen, compris entre 100 kDa et 500 kDa.

L'acide hyaluronique utilisable dans l'invention est disponible dans le commerce sous diverses formes adaptées selon les utilisations envisagées. Il peut être produit industriellement en quantités importantes par extraction à partir de tissus animaux tels que des crêtes de coq, ou par fermentation bactérienne, ou encore par procédé biotechno-logique à partir de substances végétales, par exemple du blé.

On peut utiliser par exemple de l'acide hyaluronique hydrolysé ou du sel de sodium d'acide hyaluronique, de poids moléculaire compris entre 100, de préférence 300, et 500 kDa. Les essais comparatifs effectués ont montré que les meilleurs résultats sont obtenus avec de tels acides, comme ceux disponibles dans le commerce sous la marque PrimalHyal 300 de poids moléculaire égal à 300 kDa et PrimalHyal 450 de poids moléculaire égal à 450 kDa.

Les compositions suivant la présente invention peuvent comprendre une quantité efficace de chacun des actifs ci-dessus, et par exemple entre 0,05 et 2% en poids d'acide hyaluronique, entre 0,001 et 1% en poids d'argent métallique et entre 0,1 et 15% en poids de chitosane ou de chitine, par rapport au poids total de la composition.

Le chitosane, et la chitine le cas échéant, en se combinant à l'acide hyaluronique, présente un effet de réseau réticulé protecteur qui favorise la production endogène des facteurs de croissance kératinocytaire induisant l'activité fibroblastique, augmentant la production de collagène et accélérant la différenciation des fibroblastes en myofibro-blastes.

Les compositions selon l'invention peuvent contenir en outre un ou plusieurs actifs secondaires renforçant ou complétant avantageusement l'activité de l'association d'acide hyaluronique et d'argent métallique, et compatibles, c'est-à-dire non susceptibles de réagir les uns sur les autres ou de masquer ou limiter leurs effets respectifs.

Les actifs secondaires peuvent être avantageusement choisis parmi, par exemple, un agent cicatrisant, un anti-inflammatoire, un anti-infectieux et une vitamine telle que la vitamine A ou E. L'agent cicatrisant peut être par exemple un sel de zinc, un extrait de graine de caroube riche en oligo-galactomannanes. L'anti-inflammatoire peut être un polysaccharide tel que Rhamnosoft® (Solabia) qui inhibe l'adhésion cellulaire et limite les réaction inflammatoires, ou un extrait de gomme de Boswellia serrata (Soothex®) qui agit par inhibition enzymatique de la synthèse des leukotriènes. L'anti-infectieux complémentaire peut être choisi parmi un silanol et un activateur de formation de peptide antimicrobien tel que le méthyl caproyl tyrosinate (Defensamide®).

Comme le montrent les résultats exposés ci-après, la composition de l'invention a une activité antiseptique directe sur les biofilms et augmente l'activité de l'immunité innée par les β-défensines.

Des prélèvements bactériologiques effectués sur plaie chronique chez l'animal, avant toute intervention et après application de la composition de l'invention, ont été mis en culture en milieu aérobie et en milieu anaérobie, ont été étudiés par PCR (polymerase chain reaction) microbienne, technique consistant à amplifier spécifiquement un fragment d'ADN pour le rendre détectable, mettant en évidence une diminution significative du nombre de bactéries au cours du traitement.

Des études par micro-puce ADN de l'expression génomique des β-défensines ont prouvé l'augmentation de leur sécrétion sous l'action de la composition de l'invention.

Les études effectuées ont montré que les compositions suivant l'invention ont pour effet de renforcer les défenses naturelles de la peau par libération de β-défensines-2 et -3 par les kératinocytes, qui limitent la prolifération microbienne, en particulier du fait de leur action sur les bactéries Gram-négatif aérobies et anaérobies. De plus, l'acide hyaluronique utilisé a une action anti-inflammatoire limitant les réactions douloureuses par blocage des récepteurs TRPV 1 et 3.

Les études cliniques sur des animaux souffrant de plaies traumatiques ou chroniques, ont mis en évidence une réduction considérable du temps de cicatrisation de la plaie par comparaison avec un produit de référence à base d'une association de Centella asiatica à action cicatrisante et d'huiles essentielles de romarin, de thym et de lavande à action antiseptique. La durée de cicatrisation, généralement comprise entre 7 et 15 jours pour des plaies aiguës, est diminuée de 3 à 4 jours avec la composition de l'invention. Dans le cas de plaies chroniques, un début de cicatrisation est observé dès le 5^{ème} jour avec la composition de l'invention contrairement aux compositions usuelles, telles que la sulfadiazine, qui se montrent peu efficaces. L'efficacité de la cicatrisation est confirmée par une étude de marquage des facteurs de croissance montrant une sécrétion accélérée de ces facteurs.

Les compositions selon la présente invention peuvent se présenter sous toutes les formes galéniques usuelles pour une application topique, notamment pour application topique externe. Elles peuvent se présenter par exemple sous forme de solutions aqueuses ou hydro-alcooliques, de lotions micellaires, de solutions pour pulvérisation, de shampooings, de dispersions, de sérums, de lingettes, de patchs, tulles ou pansements à libération contrôlée, de gels (aqueux, anhydres ou lipophiles), d'oléogels (gels lipidiques), de pommades, de suspensions, de dispersions vésiculaires ioniques ou non ioniques, d'émulsions liquides ou semi-liquides (par exemple un lait), solide ou semi-solide. Les émulsions peuvent être du type huile dans eau (H/E) ou eau dans huile (E/H), par exemple des gels ou des crèmes.

Les excipients et supports utilisables pour la préparation des compositions conformes à la présente invention sont ceux couramment utilisés dans les préparations à usage dermatologique ou vétérinaire, et choisis en fonction de la forme d'administration retenue. A titre d'exemple, on peut citer des émulsionnants, des épaississants, des gélifiants, des adoucissants, des conservateurs, des solubilisants, des agents de suspension ainsi que des bases lavantes et des parfums.

L'agent émulsionnant peut être choisi par exemple parmi des polymères carboxyvinyliques à haut poids moléculaire tels que le Carbopol® des polysorbates tels que le Tween 20® ou Tween 60®, un alkyl polyglucoside tel que le Montanov®, des esters de sorbitan et plus particulièrement un stéarate, un palmitate, ou un laurate de sorbitan tel que l'Arlacel®. On peut encore utiliser comme agent émulsionnant un dérivé d'acide stéarique ou palmitique, part exemple le stéarate de polyéthylène glycol, le stéarate de glycérol, le stéarate de PEG 100® (par exemple Arlacel 165®), un stéareth ou un cétéareth, un alcool gras tel qu'un alcool stéarylique, caprylique, ou cétéarylique, ou encore une silicone émulsionnable.

Les gélifiants et épaississants sont incorporés dans la composition pour en améliorer la fluidité. Ils peuvent être choisis par exemple parmi les polyacrylamides du type Carbopol, des copolymères acrylate/acide acrylique comme l'Aculyn®, des acrylates réticulés comme le Carbopol Ultrez®, des dérivés de cellulose comme l'hydroxypropyl cellulose, ou les gommes naturelles comme la gomme de xanthane.

Divers conservateurs peuvent être utilisés dans la composition de l'invention, tels que le phénoxyéthanol, le parahydroxybenzoate de méthyle (méthylparaben), le parahydroxybenzoate d'éthyle (éthylparaben), et le Seppicide HB® associant du phénoxyéthanol et des parahydroxybenzoates et présentant un spectre antimicrobien étendu.

Les agents hydratants et adoucissants utilisés dans la composition peuvent être par exemple choisis parmi le propylène glycol, la glycérine, le butylène glycol et le beurre de Karité, ainsi que les alcools gras. Un agent de suspension approprié est par exemple une argile telle que bentonite ou smectite.

Les compositions conformes à la présente invention sont préparées par les techniques usuelles, en fonction de la forme d'administration choisie, les quantités voulues d'acide hyaluronique, ou de ses sels, étant mélangées avec l'argent, et éventuellement la chitine ou le chitosane, et les supports et excipients, dans un milieu physiologiquement acceptable.

Par physiologiquement acceptable, au sens de la présente invention, on entend des supports et excipients d'un type couramment utilisé dans les compositions dermatologiques en médecine humaine et vétérinaire, neutres vis-à-vis des principes actifs utilisés, ne présentant pas d'effet toxique et n'occasionnant aucun effet secondaire néfaste sur la peau.

Par exemple, dans le cas d'une crème, on peut procéder par dispersion d'une phase grasse dans une phase aqueuse pour obtenir une émulsion huile dans eau, ou inversement pour préparer une émulsion eau dans huile, les principes actifs étant dans l'une ou l'autre phase.

La composition de l'invention, par exemple sous forme de crème, est appliquée de préférence deux à trois fois par jour sur la zone de la peau nécessitant le traitement, pendant une période de temps pouvant aller de quelques jours à quatre semaines suivant la gravité de l'affection. Elle est tout particulièrement utile pour le traitement des plaies aiguës traumatiques ou chroniques chez des animaux tels que le cheval, le chien, le chat et le lapin.

Les exemples suivants illustrent l'invention plus en détail sans en limiter la portée. Dans tous les exemples de compositions qui suivent, les parties sont exprimées en poids, sauf indication contraire.

### Exemple 1

On prépare une solution aqueuse en mélangeant à température ambiante les composants indiqués ci-après.

| | | |
|---|---|---|
| Argent métallique micronisé (10 µm) | | 0,05 |
| Acide hyaluronique PM 300 | | 0,20 |
| Chitosane | | 3,50 |
| PEG 80 sorbitan laurate | | 5,00 |
| Smectite | | 0,20 |
| Eau déminéralisée | q.s.p. | 100,00 |

L'argent métallique micronisé est un produit disponible dans le commerce sous la marque MicroSilver BG® (Impag). L'acide hyaluronique possède un poids moléculaire de 300 kDa (PrimalHyal 300®, Soliance).

### Exemple 2

Par une technique classique, on prépare une émulsion fluide (lait) ayant la composition ci-après.

| | | |
|---|---|---|
| Argent métallique micronisé (10 µm) | | 0,02 |

| | | |
|---|---|---|
| Acide hyaluronique PM 300 | | 0,10 |
| Chitosane | | 2,50 |
| Stéarate de polyéthylène glycol | | 2,00 |
| Caprylyl méthicone | | 5,50 |
| Eau déminéralisée | q.s.p. | 100,00 |

L'argent micronisé et l'acide hyaluronique sont identiques à ceux de l'Exemple 1.

### Exemple 3

On prépare une crème antiseptique par les techniques usuelles en mélangeant successivement les phases contenant les composants indiqués ci-après, les phases A et B étant mélangées à chaud (65-70°C).

| Phase A | | |
|---|---|---|
| Arlacel 165® | | 5,00 |
| Lanette 0® | | 2,00 |
| Beurre de karité | | 2,00 |
| Isononyl isonanoate | | 6,00 |

| Phase B | | |
|---|---|---|
| Eau | q.s.p. | 100,00 |
| Carbopol Ultrez® | | 0,25 |

| Phase C | | |
|---|---|---|
| Glycérine | | 5,00 |
| Argent métallique micronisé (MicroSilver) | | 0,10 |

| Phase D | | |
|---|---|---|
| Acide hyaluronique (PrimalHyal 300) | | 0,05 |
| Eau | | 10,00 |

| Phase E | | |
|---|---|---|
| Conservateur | | 0,80 |
| Parfum | | 0,25 |

Le pH de la composition est ajusté à 5,8 par addition de soude 32%.

Cette crème peut être utilisée en application sur les zones de la peau à traiter, une à deux fois par jour pendant une période de temps qui est déterminée par le praticien, et qui peut aller de une à trois semaines selon l'affection traitée.

### Exemple 4

On prépare une crème antiseptique par les techniques usuelles en mélangeant successivement les phases contenant les composants indiqués ci-après.

| Phase A | |
|---|---|
| Cetearyl Alcohol & Cetearyl Glucoside (Montanov 68®) | 4,00 |
| Cetearyl alcool | 2,50 |
| Cetiol RLF® | 3,00 |
| Stéarate de glycéryle | 3,00 |
| Huile de tournesol oléique | 6,00 |

| Phase B | |
|---|---|
| Eau | 70,56 |
| Carbopol Ultrez 21® | 0,10 |
| Gomme xanthane | 0.10 |
| Propane diol | 2,00 |
| Conservateur | 1,00 |

| Phase C | |
|---|---|
| Soude (solution aqueuse à 32%) | 0,04 |

| Phase D | |
|---|---|
| Glycérine | 2,00 |
| Argent métallique micronisé (MicroSilver) | 0,10 |

| Phase E | |
|---|---|
| Acide hyaluronique (PrimalHyal 450) | 0,10 |
| Eau | 5,00 |

| Phase F | |
|---|---|
| Chitosane succinamide | 0,50 |

Cette crème est préparée en mélangeant les phases successives comme celle de l'exemple précédent.

### Exemple 5

Une étude clinique de l'effet de l'association utilisée dans la présente invention a été faite sur 6 chiens porteurs de pathologies inflammatoires ayant entraîné des plaies aiguës traumatiques ou chroniques par lésion de grattage avec retard de cicatrisation.

Les traitements effectués antérieurement, notamment avec la sulfadiazine, s'étaient montrés inefficaces.

La composition de l'invention a été appliquée sous forme de lotion pour pulvérisation, deux fois par jour, directement sur la plaie à traiter. Les résultats ont été observés à 1 (J1), 2(J2), 3(J3), 5(J5) et 7(J7) jours après la 1^{ère} application.

Ces résultats sont regroupés dans le tableau ci-après.

| Cas | Pathologie | Observations cliniques |
|---|---|---|
| 1 | Pyodermite profonde | Cicatrisation effective à J5 |
| 2 | Retards de cicatrisation suite à une exérèse | Reprise de cicatrisation à J3 |
| 3 | Plaie de léchage suite à blessure par épillet | Amélioration à J2 Cicatrisation à J7 |
| 4 | Pyodermite interdigitée récurrente | Amélioration à J2 Cicatrisation à J7 |
| 5 | Plaie chirurgicale surinfectée par léchage après amputation pour tumeur | Amélioration à J2 Cicatrisation à 80% à J5 |
| 6 | Excoriations multiples liées à un prurit chronique atopique | Amélioration dès la première application |

Une étude des degrés de ré-épithélialisation et de contraction des plaies traumatiques a été effectuée sur les membres de six chevaux.

Sur chaque cheval, une plaie a été traitée et une autre non traitée, et l'évolution a été mesurée chaque jour pendant 14 jours, matin et soir pendant 36 heures puis une fois par jour. En fin d'étude un prélèvement bactérien a été effectué.

Le traitement a consisté en l'application de la crème de l'Exemple 4, matin et soir pendant 36 heures, puis une fois par jour.

Analyse histopathologique sur coloration Hémalun-Eosine-Safran : on observe un début de ré-épithélialisation au Jour 3 sur les plaies traitées mais pas sur les plaies témoins. Au Jour 7, les plaies sont toujours ouvertes mais rétractées, et la prolifération épithéliale est visible, avec début de kératinisation. Au Jour 14, la plaie est rétractée et la continuité de l'épipderme de surface est totalement ou presque totalement assurée par l'épithélium néoformé.

### Etude histomorphométrique :

On a utilisé un logiciel de reconnaissance des formes et de mesure des distances et des surfaces (Axiovision 4.6.1) associé au microscope. Des mesures de largeur de plaie (MES1 distance interépithéliale et MES2 distance intra-épithéliale), de longueur de l'épithélium néoformé (MES3) et de surface (SUR) de ré-épithélialisation ont été effectuées.

| | Mesure | Plaies traitées | Plaies non traitées |
|---|---|---|---|
| J2 | MES1 (µm) | 6326 | 6641 |
| | MES2 (µm) | 6326 | 6641 |
| | MES3 (µm) | 0 | 0 |
| | SUR (µm²) | 0 | 0 |
| J3 | MES1 (µm) | 5635 | 5934 |
| | MES2 (µm) | 5386 | 3358 |
| | MES3 (µm) | 343 | 500 |
| | SUR (µm²) | 8894 | 11884 |
| J7 | MES1 (µm) | 6398 | 7325 |
| | MES2 (µm) | 4817 | 6039 |
| | MES3 (µm) | 4234 | 3346 |
| | SUR (µm²) | 879989 | 651390 |
| J14 | MES1 (µm) | 3528 | 4550 |
| | MES2 (µm) | 71 | 87 |
| | MES3 (µm) | 3635 | 4905 |
| | SUR (µm²) | 922116 | 861126 |

Le tableau ci-dessous indique les rapports MES2/MES1 (2/1) et MES3/MES1 (3/1) qui sont significatifs de l'évolution de la plaie traitée ou non traitée.

| | Rapport | Plaies traitées | Plaies non traitées |
|---|---|---|---|
| J2 | 2/1 | 1 | 1 |
| | 3/1 | 0 | 0 |
| J3 | 2/1 | 0,95 | 0,56 |
| | 3/1 | 0,06 | 0,08 |
| J7 | 2/1 | 0,75 | 0,83 |
| | 3/1 | 0,66 | 0,46 |
| J14 | 2/1 | 0,02 | 0,02 |
| | 3/1 | 1,03 | 1,08 |

On observe que la plaie est plus contractée et l'épithélialisation plus avancée au Jour 7 en présence de traitement qui permet d'obtenir en 7 jours une ré-épithélialisation équivalente à celle nécessitant 14 jours sans le traitement.

Ces résultats montrent que l'association de l'invention est efficace dans le traitement des plaies aiguës ou chroniques traumatiques post brûlures, infectieuses, ou compliquant certaines dermatoses (atopie, parasitose), ou post interven-tionnelles et qu'elle apporte une solution dans les cas ou d'autres topiques se sont montrés inefficaces, notamment la sulfadiazine. Il faut souligner la rapidité de réponse (constatée le plus souvent dans les premières 48 heures) aux applications de la composition, ce qui la différencie des produits de référence comme la sulfadiazine où l'on ne constate jamais de réponse aussi rapide.

## Revendications

1. Composition dermatologique pour utilisation en médecine vétérinaire, comprenant en association au moins de l'argent métallique sous forme de particules métalliques de dimension comprise entre 1 et 20 µm, et de l'acide hyaluronique ou un de ses sels de poids moléculaire compris entre 100 kDa et 500 kDa.

2. Composition selon la revendication 1, **caractérisée en ce que** le sel d'acide hyaluronique est choisi parmi les sels de métaux alcalins et alcalino-terreux.

3. Composition selon la revendication 2, **caractérisée en ce que** le sel d'acide hyaluronique est le hyaluronate de sodium, de potassium, de calcium ou de magnésium.

4. Composition selon l'une quelconque des revendications précédentes, **caractérisée en ce qu'**elle comprend entre 0,05% et 10% en poids d'acide hyaluronique, et de préférence entre 0,1% et 10% en poids d'acide hyaluronique, par rapport au poids total de la composition.

5. Composition selon l'une quelconque des revendications précédente, **caractérisée en ce qu'**elle comprend entre 0,001% et 1% en poids d'argent métallique, par rapport au poids total de la composition.

6. Composition selon l'une quelconque des revendications précédentes, **caractérisée en ce qu'**elle comprend en outre de la chitine ou du chitosane.

7. Composition selon la revendication 6, **caractérisée en ce qu'**elle comprend entre 0,1% et 15% en poids de chitosane ou de chitine, par rapport au poids total de la composition.

8. Composition selon l'une quelconque des revendications précédentes, **caractérisée en ce que** l'argent métallique à une pureté supérieure à 99,5%.

9. Composition selon l'une quelconque des revendications précédentes, **caractérisée en ce qu'**elle est présentée sous forme pour application topique externe.

10. Composition selon l'une quelconque des revendications précédentes, **caractérisée en ce qu'**elle comprend en outre un ou plusieurs actifs secondaires choisis parmi un agent cicatrisant, un anti-inflammatoire, un anti-infectieux et une vitamine telle que la vitamine A ou E.

11. Composition selon l'une quelconque des revendications précédentes, **caractérisée en ce qu'**elle est utilisée en médecine vétérinaire dans le traitement des plaies cutanées et leur cicatrisation.

12. Composition selon l'une quelconque des revendications précédentes, **caractérisée en ce qu'**elle est utilisée pour traiter au moins un animal choisi parmi le cheval, le chien, et le chat.

## Patentansprüche

1. Dermatologische Zusammensetzung für veterinärmedizinische Verwendung, umfassend in Kombination mindestens metallisches Silber in Form von Metallpartikeln in einer Größe zwischen 1 und 20 µm und Hyaluronsäure oder eines ihrer Salze mit einem Molekulargewicht zwischen 100 kDa und 500 kDa.

2. Zusammensetzung nach Anspruch 1, **dadurch gekennzeichnet, dass** das Salz der Hyaluronsäure aus den Salzen alkalischer und erdalkalischer Metalle ausgewählt ist.

3. Zusammensetzung nach Anspruch 2, **dadurch gekennzeichnet, dass** das Salz der Hyaluronsäure das Natrium-, Kalium-, Kalzium- oder Magnesiumhyaluronat ist.

4. Zusammensetzung nach einem der vorangehenden Ansprüche, **dadurch gekennzeichnet, dass** sie zwischen 0,05 und 10 Gew.-% Hyaluronsäure und vorzugsweise zwischen 0,1 und 10 Gew.-% Hyaluronsäure in Bezug auf das Gesamtgewicht der Zusammensetzung umfasst.

5. Zusammensetzung nach einem der vorangehenden Ansprüche, **dadurch gekennzeichnet, dass** sie zwischen 0,001 und 1 Gew.-% metallisches Silber in Bezug auf das Gesamtgewicht der Zusammensetzung umfasst.

6. Zusammensetzung nach einem der vorangehenden Ansprüche, **dadurch gekennzeichnet, dass** sie ferner Chitin oder Chitosan umfasst.

7. Zusammensetzung nach Anspruch 6, **dadurch gekennzeichnet, dass** sie zwischen 0,1 und 15 Gew.-% Chitosan oder Chitin in Bezug auf das Gesamtgewicht der Zusammensetzung umfasst.

8. Zusammensetzung nach einem der vorangehenden Ansprüche, **dadurch gekennzeichnet, dass** das metallische Silber eine Reinheit von über 99,5 % hat.

9. Zusammensetzung nach einem der vorangehenden Ansprüche, **dadurch gekennzeichnet, dass** sie in Form für eine externe topische Anwendung vorliegt.

10. Zusammensetzung nach einem der vorangehenden Ansprüche, **dadurch gekennzeichnet, dass** sie ferner einen oder mehrere sekundäre Wirkstoffe umfasst, die aus einem heilenden Mittel, einem entzündungshemmenden Mittel, einem infektionshemmenden Mittel und einem Vitamin wie Vitamin A oder E ausgewählt sind.

11. Zusammensetzung nach einem der vorangehenden Ansprüche, **dadurch gekennzeichnet, dass** sie in der Veterinärmedizin für die Behandlung von Hautwunden und deren Heilung verwendet wird.

12. Zusammensetzung nach einem der vorangehenden Ansprüche, **dadurch gekennzeichnet, dass** sie zur Behandlung von mindestens einem Tier verwendet wird, ausgewählt aus dem Pferd, dem Hund und der Katze.

## Claims

1. Dermatological composition for use in veterinary medicine, comprising in association at least metallic silver, in the form of metal particles of size between 1 and 20 µm, and hyaluronic acid or one of the salts thereof having a molecular weight of between 100 kDa and 500 kDa.

2. The composition according to claim 1, **characterized in that** the hyaluronic acid salt is selected from among the salts of alkali and alkaline earth metals.

3. The composition according to claim 2, **characterized in that** the hyaluronic acid salt is sodium, potassium, calcium or magnesium hyaluronate.

4. The composition according to any of the preceding claims, **characterized in that** it comprises between 0.05 weight % and 10 weight % of hyaluronic acid, and preferably between 0.1 weight % and 10 weight % by of hyaluronic acid, relative to the total weight of the composition.

5. The composition according to any of the preceding claims, **characterized in that** it comprises between 0.001 weight % and 1 weight % of metallic silver relative to the total weight of the composition.

6. The composition according to any of the preceding claims, **characterized in that** it also comprises chitin or chitosan.

7. The composition according to claim 6, **characterized in that** it comprises between 0.1 weight % and 15 weight % by of chitosan or chitin, relative to the total weight of the composition.

8. The composition according to any of the preceding claims, **characterized in that** the metallic silver has purity higher than 99.5 %.

9. The composition according to any of the preceding claims, **characterized in that** it is presented in a form for external topical application.

10. The composition according to any of the preceding claims, **characterized in that** it also comprises one or more secondary active ingredients selected from among a healing agent, an anti-inflammatory, an anti-infective and a vitamin such as vitamin A or E.

11. The composition according to any of the preceding claims, **characterized in that** it is used in veterinary medicine in the treatment of skin wounds and healing thereof,

12. The composition according to any of the preceding claims, **characterized in that** it is used to treat at least one animal selected from among horses, dogs and cats.
